(19) Europäisches Patentamt
European Patent Office
Office européen des brevets

(11) Veröffentlichungsnummer: **0 244 717**
**A2**

# (12) EUROPÄISCHE PATENTANMELDUNG

(21) Anmeldenummer: **87106041.4**

(22) Anmeldetag: **24.04.87**

(51) Int. Cl.⁴: **C 07 D 301/26**

(30) Priorität: **07.05.86 DE 3615520**

(43) Veröffentlichungstag der Anmeldung: **11.11.87**
**Patentblatt 87/46**

(84) Benannte Vertragsstaaten: **AT BE CH DE ES FR GB IT LI NL SE**

(71) Anmelder: **BAYER AG, Konzernverwaltung RP Patentabteilung, D-5090 Leverkusen 1 Bayerwerk (DE)**

(72) Erfinder: **Häbich, Dieter, Dr., Krummacherstrasse 82, D-5600 Wuppertal 1 (DE)**
Erfinder: **Hartwig, Wolfgang, Dr., Pahlkestrasse 3, D-5600 Wuppertal 1 (DE)**

(54) **Verfahren zur Herstellung von 2,3-Epoxyamiden.**

(57) Die Erfindung betrifft ein Verfahren zur Herstellung von 2,3-Epoxyamiden der allgemeinen Formel (I)

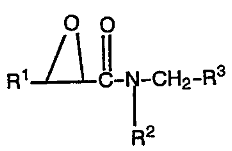

(I)

in welcher $R^1$, $R^2$, und $R^3$ die in der Beschreibung angegebene Bedeutung besitzen und die wichtige Zwischenprodukte zur Synthese von Carbapenemantibiotika sind.

EP 0 244 717 A2

 0244717

BAYER AKTIENGESELLSCHAFT 5090 Leverkusen, Bayerwerk
Konzernverwaltung RP
Patentabteilung Ad/Ke-c

# Verfahren zur Herstellung von 2,3-Epoxyamiden

Die Erfindung betrifft ein neues Verfahren zur Herstellung von teilweise bekannten 2,3-Epoxyamiden, die wichtige Zwischenprodukte zur Synthese von Carbapenemantibiotika sind.

Es ist bekannt, daß man zur Herstellung von Carbapenemantibiotika mit Hydroxyalkylseitenketten in 6-Stellung entsprechend substituierte 2,3-Epoxyamide einsetzt [H. Maruyama et al., Tetrahedron Letters 1985, 4521; H. Shiozaki et al., Heterocycles 22, 1727 (1984)].

Ebenso ist bekannt, daß solche 2,3-Epoxyamide aus den entsprechend substituierten 2-Halogen-3-hydroxyamiden durch Einwirken von starken Basen hergestellt werden. Als Basen wurden hierbei Alkalihydride, Alkaliamide, bicyclische tertiäre organische Amine, Lithiumalkylverbindungen oder sonstige Lithium-organische Verbindungen eingesetzt. Diese Basen haben mehrere Nachteile. So sind sie zum Teil schwer zugänglich und müssen direkt in der Reaktionslösung hergestellt werden. Außerdem sind sie

extrem luft- und feuchtigkeitsempfindlich, so daß ihre Handhabung, sowie die Aufarbeitung größerer Ansätze zum Teil sehr hohe sicherheitstechnische Anforderungen stellt. Die Verwendung von tertiären organischen Aminen bringt zusätzliche Probleme einer schwierigen Aufarbeitung mit sich, da sich 2,3-Epoxyamide unter sauren Bedingungen zersetzen.

Es wurde nun ein Verfahren zur Herstellung von 2,3-Epoxyamiden der allgemeinen Formel (I)

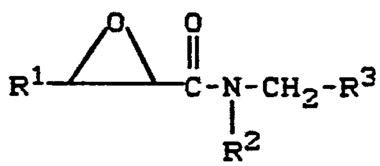

(I)

in welcher

$R^1$ - für Wasserstoff oder
- für geradkettiges oder verzweigtes $C_1$-$C_8$-Alkyl steht, das substituiert sein kann durch Phenyl oder Halogen, oder
- für $C_3$-$C_7$-Cycloalkyl,
- für Phenyl,
- für $C_1$-$C_8$-Alkoxycarbonyl oder
- für eine Gruppe der Formel

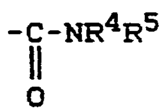

steht,

worin

$R^4$ und $R^5$ gleich oder verschieden sind und Wasserstoff, Phenyl, Benzyl, Acetyl oder gegebenenfalls durch Phenyl oder Halogen substituiertes $C_1$-$C_8$-Alkyl bedeuten,

$R^2$ - für Wasserstoff oder eine Aminoschutzgruppe steht,

und

$R^3$ - für einen die Methylengruppe aktivierenden Rest steht,

gefunden, das dadurch gekennzeichnet ist, daß man 2-Halogen-3-hydroxyamide der allgemeinen Formel (II)

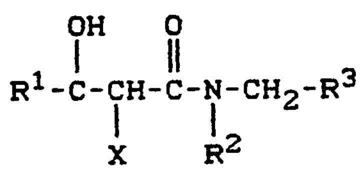 (II)

in welcher

$R^1$, $R^2$ und $R^3$ die oben angegebene Bedeutung haben,

und

X - für Fluor, Chlor, Brom oder Iod, bevorzugt für Brom steht,

in inerten Lösungsmitteln, direkt oder in Anwesenheit eines Phasentransferkatalysators mit Alkalihydroxiden umsetzt.

Überraschenderweise liefert das erfindungsgemäße Verfahren die gewünschten 2,3-Epoxyamide in guten Ausbeuten.

Das Verfahren hat den Vorteil, daß es ohne großen technischen Aufwand durchzuführen ist. Bei der erfindungsgemäßen Verwendung von Alkalihydroxiden als Basen braucht nicht auf absoluten Wasserausschluß geachtet zu werden, da keine brennbaren Gase wie Wasserstoff (bei der Verwendung von Hydriden als Basen) oder Butan (bei der Verwendung von Butyllithium als Base) entstehen können. Hierdurch wird der Aufwand für besondere zusätzliche Sicherheitsvorkehrungen vermieden. Außerdem kann das Produkt einfach durch Filtration oder Extraktion und anschließendes Eindampfen des Lösungsmittels aus der Reaktionsmischung gewonnen werden.

Die nach dem erfindungsgemäßen Verfahren hergestellten 2,3-Epoxyamide sind durch die Formel (I) allgemein definiert.

Wenn $R^2$ im Rahmen der oben angegebenen Definition für eine Aminoschutzgruppe steht, dann bevorzugt für eine in der β-Lactam-Chemie übliche Schutzgruppe aus der Reihe: 4-Methoxyphenyl, 4-Methoxymethyloxyphenyl, 4-[(2-Methoxyethoxy)methyloxy]phenyl , 3,4-Dimethoxyphenyl, Benzyl, 2-Nitrobenzyl, 4-Nitrobenzyl, 4-Methoxybenzyl, 2,4-Dimethoxybenzyl, 3,4-Dimethoxybenzyl, 2,4,6-Trimethoxybenzyl, Vinyl, Allyl, tert-Butoxycarbonyl, Benzyloxycarbonyl, 2-Nitrobenzyloxycarbonyl, 4-Nitrobenzyloxycarbonyl, Formyl, Acetyl, Chloracetyl, Trichloracetyl, Trifluoracetyl, Benzoyl, Methoxycarbonyl, Allyloxycarbonyl, 2,4-Dimethoxybenzyloxycarbonyl, 2,2-Diethoxyethyl, Methoxycarbonylmethyl, tert-Butoxycarbonylmethyl, Allyloxycarbonylmethyl, Benzoylmethyl, Bis-(4-methoxyphenyl)-methyl, Methoxymethyl, Methylthiomethyl, Methoxyethoxymethyl, 2-(Methylthiomethoxy)ethoxycarbonyl, 2-Hydroxy-2-phenylmethyl, Methoxy-(4-methoxyphenyl)methyl, Trimethyl-, Triethyl-, Triphenylsilyl, tert-Butyl-dimethylsilyl, tert-Butyl-diphenylsilyl, [2-(Trimethylsilyl)-ethoxy]methyl.

Wenn $R^2$ im Rahmen der oben angegebenen Definition für eine die Methylengruppe aktivierenden Rest steht, dann bevorzugt für einen elektronenziehenden Rest. Hierzu gehören zum Beispiel Keton- oder Estergruppen, Ether- oder Thioetherreste, Sulfinyl- oder Sulfonylgruppen, Phosphonestergruppen, die Nitril- oder Nitrogruppe, oder Acetylengruppen, die substituiert sind durch Nitro, Phenyl-, Ether-, Thioether-, Silyl-, Sulfonyl- oder Estergruppen.

Nach dem erfindungsgemäßen Verfahren werden bevorzugt 2,3-Epoxyamide der allgemeinen Formel (I)

in welcher

$R^1$ - für Wasserstoff oder
- für geradkettiges oder verzweigtes $C_1$-$C_6$-Alkyl steht, das gegebenenfalls durch Phenyl, Fluor, Chlor oder Brom substituiert ist, oder
- für $C_3$-$C_6$-Cycloalkyl,

- für $C_1$-$C_6$-Alkoxycarbonyl oder
- für eine Gruppe der Formel

$$\begin{array}{c}-C-NR^4R^5\\ \;\|\\ \;O\end{array}$$ steht,

worin

$R^4$ und $R^5$ gleich oder verschieden sind und Wasserstoff oder gegebenenfalls durch Phenyl, Fluor, Chlor oder Brom substituiertes $C_1$-$C_6$-Alkyl bedeuten,

$R^2$ - für Wasserstoff steht oder
- für eine Aminoschutzgruppe aus der Reihe 4-Methoxyphenyl, 3,4-Dimethoxyphenyl, 4-Methoxymethyloxyphenyl, 4-[(2-Methoxyethoxy)methyloxy]phenyl, Benzyl, 4-Methoxybenzyl, 2,4-Dimethoxybenzyl, 3,4-Dimethoxybenzyl, 2,4,6-Trimethoxybenzyl, Methoxy-(4-methoxyphenyl)methyl, Trimethylsilyl, Triethylsilyl, tert-Butyldimethylsilyl, tert-Butyldiphenylsilyl, Vinyl, Allyl, 2,2-Diethoxyethyl, Methoxycarbonylmethyl, tert-Butoxycarbonylmethyl, Benzoylmethyl oder 2-Hydroxy-2-phenylethyl steht

und

$R^3$ - für eine Gruppe aus der Reihe:
$-C{\equiv}C-C_6H_5$, $-C{\equiv}C-O-C_6H_5$, $-C{\equiv}C-S-C_6H_5$, $-C{\equiv}C-SO_2C_6H_5$, $-C{\equiv}C-NO_2$, $-C{\equiv}C-Si(CH_3)_3$, $-C{\equiv}C-COOCH_3$, $-C{\equiv}C-COOC_2H_5$, $-C{\equiv}C-COOC(CH_3)_3$, $-CN$, $-NO_2$, $-S-C_6H_5$, $-SO-C_6H_5$, $-SO_2-C_6H_5$, $-SO_2CH_3$, $-SO_2-C(CH_3)_3$, $-PO(OCH_3)_2$, $-PO(OC_2H_5)_2$,

$-PO(OC_6H_5)_2$, $-CO-C_6H_5$, $-COOCH_3$, $-COOC_2H_5$, $-COOC_3H_7$, $-COOCH(CH_3)_2$ oder $-COOC(CH_3)_3$, steht

hergestellt.

Besonders bevorzugt werden nach dem erfindungsgemäßen Verfahren 2,3-Epoxyamide der allgemeinen Formel (I)

in welcher

$R^1$ - für Wasserstoff oder
- für geradkettiges oder verzweigtes, gegebenenfalls durch Phenyl oder durch bis zu 3 Fluoratomen substituiertes $C_1$-$C_4$-Alkyl steht, oder
- für Cyclopropyl, Cyclopentyl, Cyclohexyl,
- für $C_1$-$C_4$-Alkoxycarbonyl oder
- für eine Gruppe der Formel

$$-\underset{\underset{O}{\|}}{C}-NR^4R^5$$ steht,

worin

$R^4$ - Wasserstoff

und

$R^5$ - gegebenenfalls durch Phenyl substituiertes $C_1$-$C_4$-Alkyl bedeutet,

$R^2$ - für Wasserstoff oder

- für eine Aminoschutzgruppe aus der Reihe 4-Methoxyphenyl, 4-Methoxybenzyl, 2,4-Dimethoxybenzyl, 3,4-Dimethoxybenzyl oder tert-Butyl-dimethylsilyl steht

und

$R^3$ - für eine Gruppe der Reihe: $-COOCH_3$, $-COOC_2H_5$, $-COOC(CH_3)_3$, $-CO-C_6H_5$, $-SO_2-C_6H_5$ oder $-SO_2-C(CH_3)_3$ steht,

hergestellt.

Verwendet man als Ausgangsprodukt (2S,3R)-N-(4-Methoxyphenyl)-N-(tert-butoxycarbonylmethyl)-2-brom-3-hydroxybutyramid so läßt sich der Reaktionsablauf durch folgendes Schema verdeutlichen:

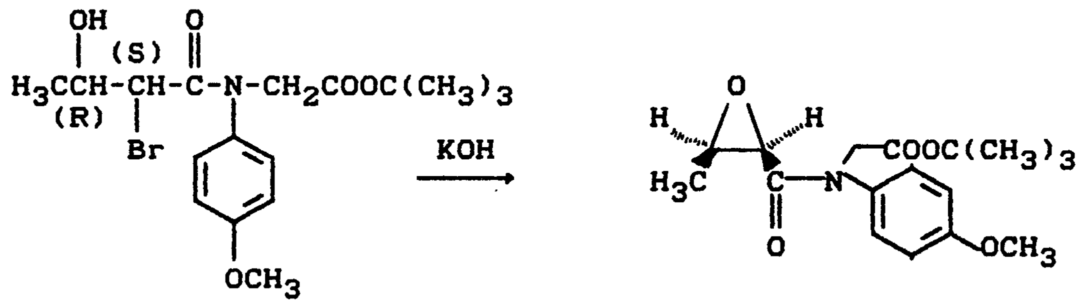


Die als Ausgangsstoffe eingesetzten 2-Halogen-3-hydroxyamide der allgemeinen Formel (II) sind bekannt oder können nach bekannten Methoden hergestellt werden [M. Shiozaki et al., Tetrahedron 40, 1795 (1984); M.Shiozaki et al., Heterocycles 22, 1727 (1984);H.Maruyama et al.,Tetrahedron Lett. 1985, 4521; M.Shiozaki et al. Bull.Chem. Soc.Jpn., 57,2135 (1984); Patentanmeldungen EP 148 128, EP 126 709, J 6 0051-171; Hanessian et al., J. Amer. Chem. Soc. 107, 1438 (1985)].

Als Lösungsmittel kommen alle inerten organischen Lösungsmittel in Frage, die sich unter den Reaktionsbedingungen nicht verändern. Hierzu gehören bevorzugt Ether wie beispielsweise Diethylether, Dioxan, Tetrahydrofuran, Dimethoxyethan, Diglyme, Triglyme oder tert-Butylmethylether, oder Halogenkohlenwasserstoffe wie beispielsweise Methylenchlorid, Chloroform, Tetrachlorkohlenstoff, Dichlorethan, 1,1,2-Trichlorethan, Dichlorethylen, Trichlorethylen, Chlorbenzol oder Dichlorbenzol, oder Kohlenwasserstoffe wie Benzol, Toluol, Xylol, Hexan, Cyclohexan oder Erdölfraktionen, oder Essigester, Acetonitril, Dimethylformamid, Hexamethylphosphorsäuretriamid, Pyridin, Picolin, Morpholin oder Piperidin, oder Gemische der genannten Lösungsmittel.

Als Alkalihydroxide können bevorzugt Lithiumhydroxid, Kaliumhydroxid oder Natriumhydroxid eingesetzt werden. Besonders bevorzugt verwendet man Kaliumhydroxid oder Natriumhydroxid.

Das erfindungsgemäß verwendete Alkalihydroxid kann entweder in fester Form, bevorzugt gepulvert, oder in Form einer wäßrigen Lösung eingesetzt werden, bevorzugt einer wäßrigen Lösung mit einer Konzentration von 10 bis 60 Gewichts-%, bevorzugt von 30 bis 55 Gewichts-% Alkalihydroxid.

Wird das Alkalihydroxid erfindungsgemäß als Feststoff eingesetzt, dann verwendet man als Lösungsmittel bevorzugt Ether wie Diethylether, Dioxan, Tetrahydrofuran, Dimethoxyethan oder tert-Butylmethylether, oder Kohlenwasserstoffe wie Benzol, Toluol, Xylol oder Cyclohexan oder Dimethylformamid, oder Gemische der genannten Lösungsmittel.
Wird das Alkalihydroxid erfindungsgemäß in Form einer wäßrigen Lösung eingesetzt, dann bevorzugt in Form einer 40 bis 50%igen Lösung. In diesem Fall werden als inertes Lösungsmittel bevorzugt Halogenkohlenwasserstoffe wie Methylenchlorid, Tetrachlorkohlenstoff, 1,2-Dichlorethan oder 1,1,2-Trichlorethan, oder Kohlenwasserstoffe wie Benzol, Toluol, Xylol oder Cyclohexan, oder Gemische der genannten Lösungsmittel eingesetzt.

Als Phasentransferkatalysatoren werden gegebenenfalls die üblichen Phasentransferkatalysatoren wie quartäre Arsonium-, Phosphonium-, Pyridinium-, Thiazolium- oder Ammoniumsalze eingesetzt.

0244717

Hierzu gehören bevorzugt:
N-Benzylcinchonidinium Chlorid,
N-Benzylcinchoninium Chlorid,
Benzyldimethylhexadecylammonium Chlorid,
Benzyldimethyltetradecylammonium Chlorid,
Benzyltributylammonium Bromid,
Benzyltributylammonium Chlorid,
Benzyltriethylammonium Bromid,
Benzyltriethylammonium Chlorid,
Benzyltriethylammonium Iodid,
Benzyltriethylammonium Tetrafluoroborat,
Benzyltrimethylammonium Bromid,
Benzyltrimethylammonium Chlorid,
Benzyltriphenylphosphonium Bromid,
Diethylmethylpropylammonium Bromid,
(-)-N,N-Dimethylephedrinium Bromid,
3,4-Dimethyl-5-(2-hydroxyethyl)thiazolium Iodid,
Dodecylethyldimethylammonium Bromid,
(-)-N-Dodecyl-N-methylephedrinium Bromid,
Ethyldimethylpropylammonium Bromid,
Ethylhexadecyldimethylammonium Bromid,
3-Ethyl-5-(2-hydroxyethyl)-4-methylthiazolium Bromid,
Ethyltriphenylphosphonium Bromid,
Hexadecylpyridinium Bromid,
Hexadecylpyridinium Chlorid,
Hexadecyltributylphosphonium Bromid,
Hexadecyltrimethylammonium Bromid,
Hexadecyltrimethylammonium Chlorid,
Methyltrioctylammonium Bromid,
Methyltrioctylammonium Chlorid,
MethyltrioctylAmmonium Iodid,
Octadecyltrimethylammonium Bromid,

Phenyltrimethylammonium Bromid,
Phenyltrimethylammonium Chlorid,
Tetrabutylammonium Bromid,
Tetrabutylammonium Chlorid,
Tetrabutylammonium Hexafluorophosphat,
Tetrabutylammonium Hydrogensulfat,
Tetrabutylammonium Hydroxid,
Tetrabutylammonium-Methansulfonat,
Tetrabutylammonium Perchlorat,
Tetrabutylammonium Tetrafluoroporat,
Tetrabutylammonium Tetraphenylborat,
Tetrabutylammonium Trifluoromethansulfonat,
Tetrabutylphosphonium Chlorid,
Tetradecyltrimethylammonium Bromid,
Tetradodecylammonium Bromid,
Tetradodecylammonium Perchlorat,
Tetraethylammonium Bromid,
Tetraethylammonium Chlorid,
Tetraethylammonium Hexafluorophosphat,
Tetraethylammonium Hydroxid,
Tetraethylammonium Perchlorat,
Tetraethylammonium Tetrafluoroburat,
Tetraethylammonium Trifluoromethansulfonat,
Tetraheptylammonium Bromid,
Tetrahexylammonium Benzoat,
Tetrahexylammonium Bromid,
Tetrahexylammonium Chlorid,
Tetrahexylammonium Hydrogensulfat,
Tetrahexylammonium Iodid,
Tetrahexylammonium Perchlorat,
Tetrakis-(decyl)-ammonium Bromid,
Tetrakis-(decyl)-ammonium Perchlorat,

Tetraoctylammonium Bromid,
Tetraoctylammonium Perchlorat,
Tetrapentylammonium Bromid,
Tetrapentylammonium Iodid,
Tetraphenylarsonium Chlorid,
Tetraphenylphosphonium Bromid,
Tetraphenylphosphonium Chlorid,
Tetrapropylammonium Bromid,
Tetrapropylammonium Hydroxid,
Tetrapropylammonium Iodid,
Tetrapropylammonium Perchlorat,
Tetrapropylammonium Tetrafluoroborat,
Tributylheptylammonium Bromid,
Tributylmethylammmonium Bromid,
Tributylmethylammonium Chlorid,
Tributylmethylammonium Hydroxid,
Tributylmethylammonium Iodid,
Tributylpentylammonium Bromid,
Tricaprylmethylammonium Chlorid ("Aliquat 336"),
Triethylammonium Bromid.

Besonders bevorzugt werden als Phasentransferkatalysatoren eingesetzt:
Tetrabutylammonium Chlorid,
Tetrabutylammonium Bromid,
Benzyltriethylammonium Chlorid,
Benzyltriethylammonium Bromid,
Benzyltributylammonium Chlorid,
Benzyltributylammonium Bromid,
Tricaprylmethylammonium Chlorid ("Aliquat 336").

Die Phasentransferkatalysatoren können auch in Form ihrer wäßrigen oder alkoholischen Lösungen eingesetzt werden. Bevorzugt werden die Phasentransferkatalysatoren erfindungsgemäß eingesetzt, wenn die Alkalihydroxide in Form ihrer wäßrigen Lösung verwendet werden.

Die Phasentransferkatalysatoren werden im allgemeinen in einer Menge von 0,01 bis 25 Mol-%, bevorzugt von 5 bis 15 Mol-% eingesetzt.
Das Alkalihydroxid kann in einem großen Überschuß verwendet werden. Im allgemeinen setzt man das Alkalihydroxid erfindungsgemäß in einer Menge von 1 Mol bis 200 Mol, bevorzugt von 1 Mol bis 100 Mol, bezogen auf 1 Mol der Ausgangsverbindung ein.

Das erfindungsgemäße Verfahren wird im allgemeinen in einem Temperaturbereich von -20°C bis +50°C, bevorzugt von 0°C bis +25°C durchgeführt.

Das erfindungsgemäße Verfahren wird im allgemeinen bei Normaldruck durchgeführt, man kann aber ebenso bei Überdruck oder Unterdruck arbeiten.

Beispiel 1

(2R,3R)-N-(tert-Butoxycarbonylmethyl)-N-(4-methoxyphenyl)-2,3-epoxybutyramid

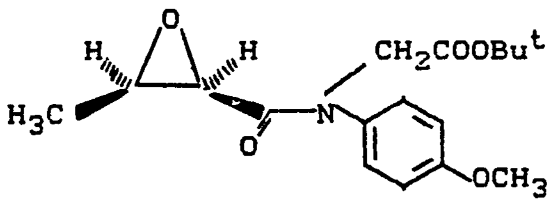

Verfahren A

Eine Lösung von 100,6 g (0,25 mol) (2S,3R)-N-(4-Methoxyphenyl)-N-(tert-butoxycarbonylmethyl)-2-brom-3-hydroxybutyramid [M. Shiozaki et al., Tetrahedron 40, 1795 (1984)] in 1000 ml Tetrahydrofuran wurde bei Raumtemperatur mit 15,5 g (0,275 mol) fein gepulvertem Kaliumhydroxid versetzt und 5,5 h bei Raumtemperatur gerührt. Nach Beendigung der Reaktion trennte man das entstandene Kaliumbromid durch Filtration ab, wusch mit Tetrahydrofuran nach und dampfte die Filtratlösung im Vakuum ein. Man erhielt 77,1 g (96% der Theorie) der Titelverbindung als Öl, $R_f$ = 0,24 (Toluol : Ethylacetat = 7:3).

IR (KBr) 1743, 1680, 1511, 1372, 1302, 1252, 1158 $cm^{-1}$.

$^1$H-NMR (250 MHz, $CDCl_3$): $\delta$ = 1,44 (d, J=6 Hz, $\underline{CH_3}CH$); 1,48 (s, $C(CH_3)_3$) zusammen 12H, 3,07 (dq, J=5 Hz, 6 Hz, 1H, H-3); 3,33 (d, J=6 Hz, 1H, H-2); 3,87 (s, 3H, $OCH_3$); 4,11 und 4,53 (AB, J=17,5 Hz, 2H, $CH_2COO$); 6,98 und 7,34 (AB, J=9,5 Hz, p-$OCH_3$ $C_6\underline{H_4}$).

Verfahren B

Eine Lösung von 230,0 g (0,57 mol) (2S,3R)-N-(4-Methoxyphenyl)-N-(tert-butoxycarbonylmethyl)-2-brom-3-hydroxybutyramid in 1500 ml Dichlormethan wurde mit 200 ml einer 50%igen (Gewichts-%) Lösung von Natriumhydroxid in Wasser versetzt und in Gegenwart von 18,4 g (0,057 mol, 10 mol-%) Tetrabutylammoniumbromid 15 min bei Raumtemperatur intensiv gerührt. Danach wurde die organische Phase im Scheidetrichter abgetrennt, mit 2 l Wasser (3 x), 1 l 0,25 N $H_2SO_4$ (1 x) und 1 l Wasser (1 x) gewaschen und über $MgSO_4$ getrocknet. Nach Abdampfen des Lösungmittels im Vakuum erhielt man 179,5 g (98% der Theorie) der Titelverbindung als Öl, $R_f$ = 0,24 (Toluol : Ethylacetat = 7:3). Die spektroskopischen Daten waren identisch mit der nach Verfahren A hergestellten Verbindung.

Beispiel 2

(2R)-N-(tert-Butoxycarbonylmethyl)-N-(4-methoxyphenyl)-2,3-epoxypropionamid

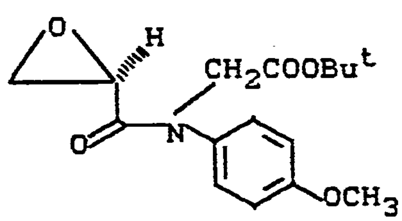

Eine Lösung von 436,8 g (0,99 mol) (2S)-N-(tert-Butoxycarbonylmethyl)-N-(4-methoxyphenyl)-2-brom-3-hydroxypropionamid in 3 l Tetrahydrofuran wurde bei Raumtemperatur mit 83,3 g (1,49 mol) gepulvertem Kaliumhydroxid versetzt und 3 h bei Raumtemperatur gerührt. Nach Beendigung der Reaktion trennte man das entstandene Kaliumbromid durch Filtration ab, spülte mit Tetrahydrofuran nach und trocknete die Filtratlösung kurz über $MgSO_4$. Nach Abdampfen des Lösungmittels im Vakuum erhielt man 295,2 g (97% der Theorie) der Titelverbindung als Öl, $R_f$ = 0,32 (Toluol : Ethylacetat = 7:3).

$^1$H-NMR (300 MHz, $CDCl_3$): δ = 1,45 (s, 9H, $C(CH_3)_3$); 2,74 (dd, J=7 Hz, 5 Hz, 1H, Epoxid-H); 3,01 (dd, J=7 Hz, 3 Hz, 1H Epoxid-H); 3,25 (dd, J=5 Hz, 3 Hz, 1H, Epoxid-H); 3,84 (s, 3H, $OCH_3$); 4,15 und 4,43 (AB, J=18 Hz, 2H, $CH_2COO$); 6,94 und 7,33 (AB, J=9 Hz, 4H, p-$OCH_3$-$C_6\underline{H_4}$).

Beispiel 3

(2R,3S)-N-(tert-Butoxycarbonylmethyl)-N-(4-methoxyphenyl)-3-methoxycarbonyl-2,3-epoxypropionamid

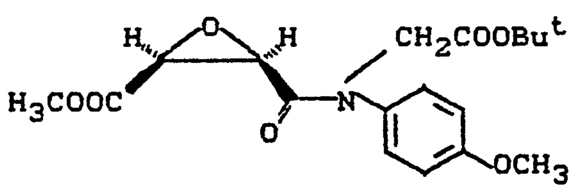

Wie für Verfahren B aus Beispiel 1 beschrieben, erhielt man aus 9,8 g (22 mmol) (2S,3R)-N-(tert-Butoxycarbonylmethyl)-N-(4-methoxyphenyl)-2-brom-3-hydroxy-3-methoxycarbonylpropionamid nach 17 min bei Raumtemperatur die Titelverbindung als farblose Kristalle, Schmp.: 90°C, $R_f$ = 0,17 (Toluol : Ethylacetat = 4:1).

IR ($CHCl_3$) 1737 (C=O, Ester),1681 (C=O, Amid) 1509 $cm^{-1}$.

$^1$H-NMR (250 MHZ, $CDCl_3$): δ = 1,44 (s, 9H, $CH_3$-C); 3,46 und 3,51 (AB, J=4,5 Hz, 2H, Oxiran-H); 3,82 (s,

6H, $OCH_3$, $COOCH_3$); 4,08 und 4,36 (AB, J=16 Hz, $CH_2COO$); 6,91 und 7,31 (AB, J=9 Hz, 4H, p-$OCH_3$ $C_6H_4$).

Beispiel 4

(2R,3R)-N-(Benzoylmethyl)-N-(4-methoxyphenyl)-2,3-epoxy-butyramid

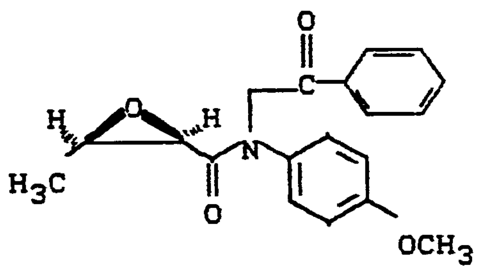

Man verfuhr analog Verfahren B, Beispiel 1.
Eingesetzt wurden 50 g (0,123 mol) (2S,3R)-N-(4-Methoxy-phenyl)-N-(benzoylmethyl)-2-brom-3-hydroxybutyramid [Hanessian et al., J.Am.Chem.Soc. 107, 1438 (1985)]. Man erhielt 38,4 g (96% der Theorie) der Titelverbindung mit $R_f$ = 0,31 (Cyclohexan : Essigester = 1:2).
$^1$H-NMR (250 MHz, $CDCl_3$): $\delta$ = 1,48 (d, J=6 Hz, 3H, $CH_3CH$); 3,09 (dq, J=6 Hz, J=5 Hz, 1H, C(3)-H); 3,39 (d, J=5 Hz, 1H, C(2)-H); 3,82 (s, 3H, $OCH_3$); AB-Signal ($\delta_A$ = 4,87, $\delta_B$ = 5,43, $J_{AB}$ = 16 Hz, 2H, $CH_2$-C=O); AB-Signal ($\delta_A$ = 6,92, $\delta_B$ = 7,96, J=9 Hz, 4H, p-$OCH_3$-$C_6H_4$); 7,28 - 7,65 (m, 5H, $C_6H_5$).

Beispiel 5

(2R,3R)-N-(Methoxycarbonylmethyl)-2,3-epoxybutyramid

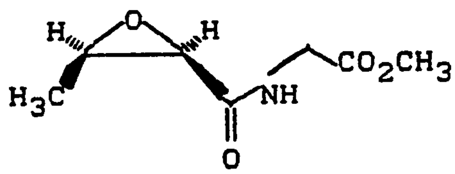

Man verfuhr analog Verfahren B, Beispiel 1. Eingesetzt wurden 25 g (0,098 mol) (2S,3R)-N-(Methoxycarbonylmethyl)-2-brom-3-hydroxybutyramid. Man erhielt 13,9 g (82% der Theorie) der Titelverbindung mit $R_f$ = 0,39 (Essigester).

$^1$H-NMR (300 MHz, $CDCl_3$): $\delta$ = 1,41 (d, J=6 Hz, 3 Hz, $CH_3$); 3,32 (dq, J=5 Hz, J=6 Hz, 1H, C(3)-H); 3,54 (d, J=5 Hz, 1H, C(2)-H); 3,78 (s, 3H, $OCH_3$); ABX-Signal ($\delta_A$ = 3,95, $\delta_B$ = 4,23, $J_{AB}$ = 18,5 Hz, $J_{AM}$ = $J_{BM}$ = 6 Hz, 2H, $-CH_2-$); 6,72 (br, 1H, NH).

Beispiel 6

(2R,3R)-N-(Methoxycarbonylmethyl)-N-(2,4-dimethoxyphenylmethyl)-2,3-epoxybutyramid

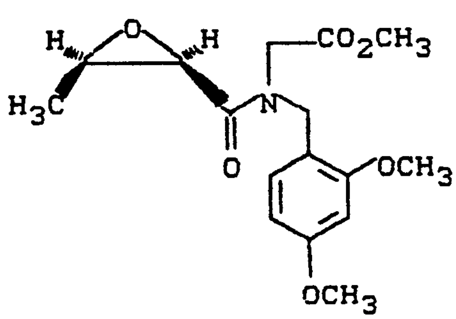

Man verfuhr analog Verfahren B, Beispiel 1. Eingesetzt wurden 50 g (0,124 mol) (2S,3R)-N-(Methoxycarbonylmethyl)-N-(2,4-dimethoxyphenylmethyl)-2-brom-3-hydroxybutyramid. Man erhielt 38,8 g (97% der Theorie) der Titelverbindung mit $R_f$ = 0,25 (Cyclohexan : Essigester = 1:2).

$^1$H-NMR (250 MHz, $CDCl_3$): δ = 1,40 (d, J=6 Hz, 3H, $CH_3$); 3,40 (d, q, J=5 Hz, J06 Hz, 1H, C(3)-H); 3,59 8d, J=5 Hz, 1H, C(2)-4); 3,73, 3,84, 3,86 (je s, 9H, $OCH_3$); AB-Signal ($\delta_A$ = 4,60 ($\delta_B$ = 4,58, J=17,5 Hz; 2H, $CH_2CO_2$); AB-Signal ($\delta_A$ = 4,60, $\delta_B$ = 4,74, J=17 Hz, 2H, $\underline{CH_2}$-$C_6H_3(OCH_3)_2$);AB-Signal ($\delta_A$ = 6,47, $\delta_B$ = 7,10, J=9 Hz, 2H, C(5), C(6)-$C_6H_3(OCH_3)_2$); 6,51 (s, 1H, C(3)-$C_6H_3(OCH_3)_2$).

## Patentansprüche

1. Verfahren zur Herstellung von 2,3-Epoxyamiden der allgemeinen Formel (I)

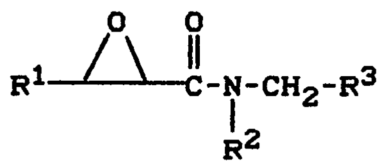

(I)

in welcher

$R^1$ - für Wasserstoff oder
- für geradkettiges oder verzweigtes $C_1$-$C_8$-Alkyl steht, das gegebenenfalls substituiert sein kann durch Phenyl oder Halogen, oder
- für $C_3$-$C_7$-Cycloalkyl,
- für Phenyl,
- für $C_1$-$C_8$-Alkoxycarbonyl oder
- für eine Gruppe der Formel

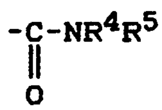

steht,

worin

$R^4$ und $R^5$ gleich oder verschieden sind und Wasserstoff, Phenyl, Benzyl, Acetyl oder gegebenenfalls durch Phenyl oder Halogen substituiertes $C_1$-$C_8$-Alkyl bedeuten,

$R^2$ - für Wasserstoff oder eine Aminoschutzgruppe steht,

und

$R^3$ - für einen die Methylengruppe aktivierenden Rest steht,

dadurch gekennzeichnet, daß man 2-Halogen-3-hydroxyamide der allgemeinen Formel (II)

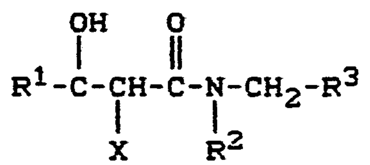 (II)

in welcher

$R^1$, $R^2$ und $R^3$ die oben angegebene Bedeutung haben,

und

X - für Fluor, Chlor, Brom oder Iod, bevorzugt für Brom steht,

in inerten Lösungsmitteln, direkt oder in Anwesenheit eines Phasentransferkatalysators mit Alkalihydroxiden umsetzt.

2. Verfahren zur Herstellung von 2,3-Epoxyamiden der allgemeinen Formel (I) gemäß Anspruch 1, dadurch gekennzeichnet, daß man 2-Halogen-3-hydroxyamide der Formel (II) einsetzt, in der X die in Anspruch 1 angegebene Bedeutung hat und

$R^1$ - für Wasserstoff oder
- für geradkettiges oder verzweigtes $C_1$-$C_6$-Alkyl steht, das gegebenenfalls durch Phenyl, Fluor, Chlor oder Brom substituiert ist, oder
- für $C_3$-$C_6$-Cycloalkyl,
- für $C_1$-$C_6$-Alkoxycarbonyl oder
- für eine Gruppe der Formel

$$-\underset{\underset{O}{\parallel}}{C}-NR^4R^5$$ steht,

worin

$R^4$ und $R^5$ gleich oder verschieden sind und Wasserstoff oder gegebenenfalls durch Phenyl, Fluor, Chlor oder Brom substituiertes $C_1$-$C_6$-Alkyl bedeuten,

$R^2$ - für Wasserstoff steht oder
- für eine Aminoschutzgruppe aus der Reihe 4-Methoxyphenyl, 3,4-Dimethoxyphenyl, 4-Methoxymethyloxyphenyl, 4-[(2-Methoxyethoxy)-methyloxy]phenyl, Benzyl, 4-Methoxybenzyl, 2,4-Dimethoxybenzyl, 3,4-Dimethoxybenzyl, 2,4,6-Trimethoxybenzyl, Methoxy-(4-methoxyphenyl)methyl, Trimethylsilyl, Triethylsilyl, tert-Butyldimethylsilyl, tert-Butyl-diphenylsilyl, Vinyl, Allyl, 2,2-Diethoxyethyl, Methoxycarbonylmethyl, tert-Butoxycarbonylmethyl, Benzoylmethyl oder 2-Hydroxy-2-phenylethyl steht

und

$R^3$ - für eine Gruppe aus der Reihe:
$-C \equiv C-C_6H_5$, $-C \equiv C-O-C_6H_5$, $-C \equiv C-S-C_6H_5$, $-C \equiv C-SO_2C_6H_5$, $-C \equiv C-NO_2$, $-C \equiv C-Si(CH_3)_3$, $-C \equiv C-COOCH_3$, $-C \equiv C-COOC_2H_5$, $-C \equiv C-COOC(CH_3)_3$, $-CN$, $-NO_2$, $-S-C_6H_5$, $-SO-C_6H_5$, $-SO_2-C_6H_5$, $-SO_2CH_3$, $-SO_2-C(CH_3)_3$, $-PO(OCH_3)_2$, $-PO(OC_2H_5)_2$, $-PO(OC_6H_5)_2$, $-CO-C_6H_5$, $-COOCH_3$, $-COOC_2H_5$, $-COOC_3H_7$, $-COOCH(CH_3)_2$ oder $-COOC(CH_3)_3$, steht

3. Verfahren nach den Ansprüchen 1 und 2, dadurch gekennzeichnet, daß man für die Umsetzung Alkalihydroxide verwendet.

4. Verfahren zur Herstellung von 2,3-Epoxyamiden der allgemeinen Formel (I) gemäß Anspruch 1, dadurch gekennzeichnet, daß man 2-Halogen-3-hydroxyamide der Formel (II) einsetzt, in der X die in Anspruch 1 angegebene Bedeutung hat und

$R^1$ - für Wasserstoff oder
- für geradkettiges oder verzweigtes, gegebenenfalls durch Phenyl oder durch bis zu 3 Fluoratomen substituiertes $C_1$-$C_4$-Alkyl steht, oder

- für Cyclopropyl, Cyclopentyl, Cyclohexyl,
- für $C_1$-$C_4$-Alkoxycarbonyl oder
- für eine Gruppe der Formel

$$-\underset{\underset{O}{\|}}{C}-NR^4R^5$$ steht,

worin

$R^4$ - Wasserstoff

und

$R^5$ - gegebenenfalls durch Phenyl substituiertes $C_1$-$C_4$-Alkyl bedeutet,

$R^2$ - für Wasserstoff oder
- für eine Aminoschutzgruppe aus der Reihe 4-Methoxyphenyl, 4-Methoxybenzyl, 2,4-Dimethoxybenzyl, 3,4-Dimethoxybenzyl oder tert-Butyl-dimethylsilyl steht

und

$R^3$ - für eine Gruppe der Reihe: $-COOCH_3$, $-COOC_2H_5$, $-COOC(CH_3)_3$, $-CO-C_6H_5$, $-SO_2-C_6H_5$ oder $-SO_2-C(CH_3)_3$ steht.

5. Verfahren nach den Ansprüchen 1 bis 3, dadurch gekennzeichnet, daß man für die Umsetzung Lithium-, Kalium- oder Natriumhydroxid in fester Form oder

als wäßrige Lösung mit einer Konzentration von 10 bis 60 Gew.-%, insbesondere 30 bis 55 Gew.-%, Alkalihydroxid verwendet.

6. Verfahren nach Anspruch 5, dadurch gekennzeichnet, daß man als Phasentransferkatalysator

N-Benzylcinchonidinium Chlorid,
N-Benzylcinchoninium Chlorid,
Benzyldimethylhexadecylammonium Chlorid,
Benzyldimethyltetradecylammonium Chlorid,
Benzyltributylammonium Bromid,
Benzyltributylammonium Chlorid,
Benzyltriethylammonium Bromid,
Benzyltriethylammonium Chlorid,
Benzyltriethylammonium Iodid,
Benzyltriethylammonium Tetrafluoroborat,
Benzyltrimethylammonium Bromid,
Benzyltrimethylammonium Chlorid,
Benzyltriphenylphosphonium Bromid,
Diethylmethylpropylammonium Bromid,
(-)-N,N-Dimethylephedrinium Bromid,
3,4-Dimethyl-5-(2-hydroxyethyl)thiazolium Iodid,
Dodecylethyldimethylammonium Bromid,
(-)-N-Dodecyl-N-methylephedrinium Bromid,
Ethyldimethylpropylammonium Bromid,
Ethylhexadecyldimethylammonium Bromid,
3-Ethyl-5-(2-hydroxyethyl)-4-methylthiazolium Bromid,
Ethyltriphenylphosphonium Bromid,
Hexadecylpyridinium Bromid,
Hexadecylpyridinium Chlorid,

Hexadecyltributylphosphonium Bromid,
Hexadecyltrimethylammonium Bromid,
Hexadecyltrimethylammonium Chlorid,
Methyltrioctylammonium Bromid,
Methyltrioctylammonium Chlorid,
MethyltrioctylAmmonium Iodid,
Octadecyltrimethylammonium Bromid,
Phenyltrimethylammonium Bromid,
Phenyltrimethylammonium Chlorid,
Tetrabutylammonium Bromid,
Tetrabutylammonium Chlorid,
Tetrabutylammonium Hexafluorophosphat,
Tetrabutylammonium Hydrogensulfat,
Tetrabutylammonium Hydroxid,
Tetrabutylammonium-Methansulfonat,
Tetrabutylammonium Perchlorat,
Tetrabutylammonium Tetrafluoroporat,
Tetrabutylammonium Tetraphenylborat,
Tetrabutylammonium Trifluoromethansulfonat,
Tetrabutylphosphonium Chlorid,
Tetradecyltrimethylammonium Bromid,
Tetradodecylammonium Bromid,
Tetradodecylammonium Perchlorat,
Tetraethylammonium Bromid,
Tetraethylammonium Chlorid,
Tetraethylammonium Hexafluorophosphat,
Tetraethylammonium Hydroxid,
Tetraethylammonium Perchlorat,
Tetraethylammonium Tetrafluoroburat,
Tetraethylammonium Trifluoromethansulfonat,
Tetraheptylammonium Bromid,

Tetrahexylammonium Benzoat,
Tetrahexylammonium Bromid,
Tetrahexylammonium Chlorid,
Tetrahexylammonium Hydrogensulfat,
Tetrahexylammonium Iodid,
Tetrahexylammonium Perchlorat,
Tetrakis-(decyl)-ammonium Bromid,
Tetrakis-(decyl)-ammonium Perchlorat,
Tetraoctylammonium Bromid,
Tetraoctylammonium Perchlorat,
Tetrapentylammonium Bromid,
Tetrapentylammonium Iodid,
Tetraphenylarsonium Chlorid,
Tetraphenylphosphonium Bromid,
Tetraphenylphosphonium Chlorid,
Tetrapropylammonium Bromid,
Tetrapropylammonium Hydroxid,
Tetrapropylammonium Iodid,
Tetrapropylammonium Perchlorat,
Tetrapropylammonium Tetrafluoroborat,
Tributylheptylammonium Bromid,
Tributylmethylammmonium Bromid,
Tributylmethylammonium Chlorid,
Tributylmethylammonium Hydroxid,
Tributylmethylammonium Iodid,
Tributylpentylammonium Bromid,
Tricaprylmethylammonium Chlorid ("Aliquat 336")
oder
Triethylammonium Bromid.

einsetzt.

7. Verfahren nach Anspruch 5, dadurch gekennzeichnet, daß man als Phasentransferkatalysator

Tetrabutylammonium Chlorid,
Tetrabutylammonium Bromid,
Benzyltriethylammonium Chlorid,
Benzyltriethylammonium Bromid,
Benzyltributylammonium Chlorid,
Benzyltributylammonium Bromid oder
Tricaprylmethylammonium Chlorid ("Aliquat 336"),

einsetzt.

8. Verfahren nach Ansprüchen 1 bis 7, dadurch gekennzeichnet, daß man den Phasentransferkatalysator in einer Menge von 0,01 bis 25 Mol-%, bezogen auf die Verbindung der Formel (II), und das Alkalihydroxid in einer Menge von 1 bis 200 Mol, bezogen auf 1 Mol der Verbindung der Formel (II) einsetzt.

9. Verfahren nach Ansprüchen 1 bis 8, dadurch gekennzeichnet, daß man die Reaktion bei -20° C bis +50° C durchführt.

10. Verfahren nach Ansprüchen 1 bis 8, dadurch gekennzeichnet, daß man die Reaktion bei 0° C bis +25° C durchführt.